(19) Europäisches Patentamt
European Patent Office
Office européen des brevets

(11) Publication number: **0 089 229**
**B1**

(12) # EUROPEAN PATENT SPECIFICATION

(45) Date of publication of patent specification: **15.10.86**

(21) Application number: **83301424.4**

(22) Date of filing: **15.03.83**

(51) Int. Cl.⁴: **C 07 D 493/14,** A 61 K 31/36 // (C07D493/14, 317:00, 311:00, 307:00)

(54) **Physiologically active pterocarpan compounds, methods for the isolation thereof and therapeutically active compositions containing them.**

(30) Priority: **15.03.82 US 357805**
**15.03.82 US 358191**

(43) Date of publication of application:
**21.09.83 Bulletin 83/38**

(45) Publication of the grant of the patent:
**15.10.86 Bulletin 86/42**

(84) Designated Contracting States:
**AT BE CH DE FR GB IT LI LU NL SE**

(56) References cited:
**HETEROCYCLES, vol. 15, no. 2, 1981, pages 1163-1170, V.S. KAMAT et al.: "Antimicrobial agents from an East African medicinal plant"**

**TETRAHEDRON LETTERS, vol. 23, no. 38, September 1982, pages 3859-62, Pergamon Press Ltd., Oxford, GB, MASAJI ISHIGURO et al.: "Synthesis of (+-) -cabenegrins A-I and A-II"**

(73) Proprietor: **RICHTER GEDEON VEGYESZETI GYAR R.T.**
**Gyömröi ut 19-21**
**H-1103 Budapest X (HU)**

(72) Inventor: **Darko, Laszlo L.**
**29 Orchard Drive**
**Redding Connecticut (US)**
Inventor: **Nakanishi, Koji**
**560 Riverside Drive**
**New York New York 10027 (US)**
Inventor: **Nakagawa, Masachi**
**5-28-19 Sakurai Shimamoto-cho**
**Nishima-gun 618 Osaka (JP)**

(74) Representative: **Pett, Christopher Phineas et al**
**Frank B. Dehn & Co. Imperial House**
**15-19 Kingsway**
**London WC2B 6UZ (GB)**

(56) References cited:
**TETRAHEDRON LETTERS, vol. 23, no. 38, September 1982, pages 3855-3858, Pergamon Press Ltd., Oxford, GB, MASASHI NAKAGAWA et al.: "Structures of cabenegrins A-I and A-II, potent anti-snake venoms"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 089 229 B1**

## Description

This invention relates to physiologically active pterocarpan-compounds, methods for the isolation thereof and therapeutically active compositions containing them. More particularly it relates to products of manufacture which are substantially pure crystalline pterocarpan compounds which have been isolated from the crude extracts of natural products and to methods for isolation of the compounds.

It is known that the aqueous alcoholic extract of the root of the South American cabeca de negra tree has been available to planatation workers in the upper Amazon jungle as an oral antidote against snake and spider venoms. About ten varieties of the species cabeca de negra are known in South America. Neither the nature of the active components nor studies of the side effects, pharmacological activity, stability and the like are known to have been published on the compounds of the invention. Pterocarpans possessing antimicrobial properties have been identified in the literature, see Heterocycles, Vol. 15, 1163 [1981].

In one aspect, the invention relates to pterocarpan compounds having the formula (I)

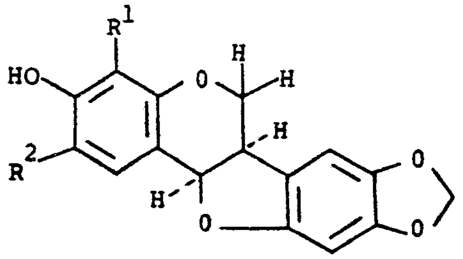

(I)

wherein $R^1$ represents a hydrogen atom and $R^2$ represents a $-CH_2CH_2-CH(CH_3)-CH_2OH$ group, or $R^1$ represents a

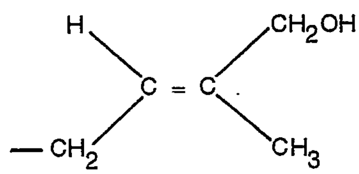

group

and $R^2$ represents a hydrogen atom, together with all optical isomers and racemic mixtures thereof.

We have been able to isolate such compounds in substantially pure crystalline form. For convenience, the compounds of the above structure will be referred to hereinafter as cabenegrin I [3-hydroxy-4-(3-hydroxy-methyl-butene-2)-8,9-methylenedioxy-pterocarpan] and cabenegrin II [2-(3-hydroxymethyl-butyl)-3-hydroxy-8,9-methylenedioxy-pterocarpan], respectively. The invention also relates to a step-wise method employed to recover and isolate the essentially pure — 95—100% — crystalline products referred to as cabenegrin I and cabenegrin II.

The compounds cabenegrin I and cabenegrin II have pharmaceutical utility in the treatment of mammals, including man, that have been envenomated by poisonous snakes and insects. These compounds have been found to acts as potent antidotes against effects of snake and insect toxins. The compounds of the invention are also useful in treating the effects of other known organic toxins such as *E. coli* endotoxin and that produced by *Clostridium botulinum*, commonly referred to as botulism or food poisoning. Pharmaceutical utility is generally indicated for the treatment of the effects in mammals of toxins produced by pathogenic bacteria (endotoxins and exotoxins) which attack the nervous system of the victim and in particular where paralysis of the respiratory system is manifested. Treatment against the effects of cardio-vascular toxins is also indicated.

The compounds can be administered orally e.g. in liquid form as a suspension or solubilized in a compatible pharmaceutical carrier. Pure crystalline material can be administered orally in capsule or tablet form when compounded with suitable pharamaceutical carriers. Alternatively, either of the compounds cabenegrin I or II can be dissolved in a suitable liquid pharmaceutical carrier and the solution administered intravenously by syringe, or by catheter where monitoring equipment is available to determine the effect of the therapy. If desired, the compounds of the invention can be mixed together in various proportions, and the mixture prepared as described above with suitable pharmaceutical carriers.

A crude extract from which the compounds of the invention may be obtained may be prepared as follows:

Approximately ½ kilo of the washed root of the cabeca de negra tree is chopped into small pieces which may be macerated, pulverized or otherwise treated to break down the fibrous structure. This step can be accomplished in a blender or laboratory homogenizer. Roots evidencing mould or fungus should not be used. The pulverized root is placed in a large glass beaker or other suitable vessel which may be covered and subsequently stirred. A sufficient quantity of the mixture of water and aliphatic alcohol having 1—4 carbon atoms, preferably ethanol:water 77:23 is added to cover the pulverized root and briskly stirred for a few minutes. The vessel containing the aqueous ethanol and root is allowed to stand at ambient temperatures for at least 48 hours, with occasional stirring. At the end of this period, the aqueous alcoholic solution comprising the crude extract is separated from the root by any convenient means, for example, by pouring through medium filter paper. The pulverized root is discarded. The filtered solution is the color of strong tea.

The following procedure may then be employed to isolate the active compounds from the crude extract as prepared above.

The aqueous ethanol crude extract (135 ml) was concentrated by gently warming under vacuum to obtain 1.2 grams of a brown oily residue. This concentrated material was treated with 50% aqueous methanol and the solution was extracted by vigorous shaking with an aliphatic hydrocarbon having 5—8 carbon atoms, preferably with hexane. The hexane layer was separated and discarded. The water layer was extracted with ether by vigorous shaking. The ether layer and the water layer was separated, the ether layer (640 milligrams) being set aside. The water layer was extracted with n-butyl alcohol by vigorous shaking. The n-butyl alcohol layer was separated and set aside for a possible investigation, and the water layer was discarded.

The ether layer extracted above is separated using well-known chromatographic methods. Preferably, the ether layer extracted above is subjected in a first step to high pressure liquid chromatography (HPLC) on Sephadex LH-20 and as a second step on silica gel using aqueous methanol as the eluting solvent. This procedure results in two fractions and the first is further separated by HPLC employing Partisil-10 eluted with 3% methanol in methylene chloride to yield pure solid compounds.

The compound identified as cabenegrin I is recovered as a white crystalline material in a yield of 44 mg. A sharp melting point of 167—168°C is obtained and analysis shows the composition to be $C_{21}H_{20}O_6$. The Rf value of this compound on thin layer chromatography employing silica gel CO/Kieselguhr F-254 and benzene/ethyl acetate/methanol (15/4/1) was 0.53.

The U.V., C.D., and I.R. of compound I are as follows:

UV (in MeOH): 209 nm (ε 75,000), 233 nm shoulder, (ε 24,000), 309 nm (ε 13,000). CD (in MeOH): 213 nm (Δε −25.38), 220 nm (Δε −2.00), 238 nm (Δε −9.84); 302 nm (Δε +3.15). IR (in $CHCl_3$): 3550 $cm^{-1}$, 1600 $cm^{-1}$, 1113 $cm^{-1}$, 925 $cm^{-1}$.

In addition to these spectral data, the following $^1$H-NMR, $^{13}$C-NMR, and MS (EI) data were measured concering cabenegrin I:

$^1$H-NMR:
1.83 (3H, d, 1 Hz, 4'—$CH_3$);
3.40 (2H, br, d, 7 Hz, C 1');
3.40 (2H, ddd, 7, 7.5 Hz, C 6a);
3.61 (2H, d, d, 11.7 Hz, c 6β);
3.90 (2H, —$OCH_2$O);
3.99 (2H, br, s, —$CH_2$—OH);
4.28 (1H, dd, 11.5 Hz, C 6α);
5.45 (1H, br, t, 7 Hz, C 2');
5.45 (1H, d, 7 Hz, C 11a);
6.41 (1H, s C 10);
6.52 (1H, d, 9 Hz, C 2);
6.70 (1H, s, C 7);
7.23 (1H, d, 9 Hz, C 1) ppm.

$^{13}$C—NMR:
13.74 (4'—$CH_3$); 21.88 (C 1'); 40.16 (C 6a);
66.72 (C 6); 68.72 (—$CH_2$—OH); 79.10 (C 11a);
93.77 (C 10); 104.73 (C 7); 109.6 (C 2);
112.63 (C 4 or C 1a); 115.01 (C 4 or C 1a);
118.04 (C 6b); 123.49 (C 2'); 129.15 (C 1);
136.07 (C 3'); 141.67 (C 8); 148.06 (C 9);
154.20 (C 10a or C 4a); 154.20 (C 10a or C 4a);
155.08 (C 3) ppm.

MS (18 eV):
$M^+$ = 368, 50%
m/e = 350, 100%; 335, 95%; 335, 95%;
176, 65%; 161, 90%.

The second sample of material recovered from the HPLC described above comprised an oily mixture of approximately 10 mg. This second active fraction, which had been obtained from the HPLC of the ether layer described above, is treated as follows to obtain the compound cabenegrin II. The mixture was subjected to further HPLC employing μ-Bondapack C18 and methanol/acetonitrile/$H_2O$/n-PrOH(71/71/59/2). The compound (II) was obtained in essentially pure — 95—100% — crystalline form in a yield of about 1 mg. The structure of this compound is based on the following physical constants, and analysis (MS) shows the composition to be $C_{21}H_{22}O_6$.

$^1$H-NMR:
0.69: (3H, d, 5.8 Hz 3'—$CH^3$);
2.50 (2H, m, C 1');
2.67 (2H, m, C 1');
2.94 (1H, ddd, 9.1, 6.3, 4.9 Hz, C 6a);
3.10 (2H, dd, 8.8, 2.7 Hz, —$CH_2$—OH);
3.18 (2H, dd, 8.8, 4.4 Hz, —$CH_2$—OH);
3.50 (1H, dd, 9.1, 9.1. Hz, C 6β);
3.86 (1H, dd, 9.1, 4.9 Hz, C 6α);
5.27 (1H, d, 6.3 Hz, C 11a);
5.30 (2H, —$OCH_2$O);
5.33 (2H, —$OCH_2$O—);
6.36 (1H, C 10);
6.52 (1H, C 4);
6.53 (1H, C 7);
7.08 (1H, C 1) ppm.

Uv (in MeOH): 204 nm (ε 116,000), 230 nm (ε 8,000), 292 nm (ε 9,400), 308 nm (ε 11,800).

CD (in MeOH): 237 nm (Δε −6.68), 280 nm (Δε −0.46), 299 nm (Δε −1.72).

The structure of this compound is shown by NMR data to be a 3:1 mixture of epimers at C-3' and the invention includes the individual optical issomers together with racemic mixtures within its scope. The structures of both compounds cabenegrin I and cabenegrin II have been confirmed by synthesis of their respective racemates.

The new compounds produced by the method of the invention are suitable for making therapeutically active compositions, which are antidotes for treating the effects of poisonous snake and insect bites in mammals, including man.

They are also suitable for the treatment of pathogenic bacterial toxins as *E. coli* endotoxins, botulism and others which exhibit central nervous system effects and related respiratory paralysis, and to the treatment of the effects of cardiovascular toxins on mammals.

As for the new compounds obtained by the above extraction methods, the presence of physiologically active compounds was determined by *in vivo* tests employing mice. Test animals were Swiss Webster white mice, mixed sexes, weighing from 25—30 grams. Each group of test animals was envenomated with two and one-half times the lethal does of snake venom from the Fer de Lance (*Bothrops atrox*) by intraperitoneal injection. In the absence of treatment, envenomated animals succumbed within a few minutes.

Concentrates of compounds of the invention obtained from each of the fractions of the above extraction scheme were tested for antidotal activity by injecting the mice immediately after envenomation with an aqueous ethanol solution (77.23) of the material isolated from each fractions. Each animal was treated with 0.25 ml of the respective solutions. On the basis of this protocol, the minimum dosage for survival against the Fer de Lance venom was 2.8 mg/kg of cabenegrin I and 2.0 mg/kg of cabenegrin II.

Toxicological studies indicate that administration of aqueous ethanolic solutions of cabenegrin I and cabenegrin II to healthy test animals produce no significant changes in vital physiological functions. Administration can be by intravenous or intramuscular injection, or orally via a stomach tube. No significant change is noted in arterial blood pressure, heart rate, respiration, EKG or central venous pressure at any time following administration of compounds I and II to normal healthy (i.e. non-envenomated) animals.

Administration does not significantly alter resting action potentials, end plate potentials, nerve impulse transmission, neuro-muscular function or brain wave activity in experimental animals.

1. Antidotal effects against snake venom in dogs.

Envenomation by a lethal dose of snake venom, such as *Bothrops atrox, Crotalus adamanteus* or *Crotalus atrox*, produces a precipitous fall in arterial blood pressure, a decrease of heart rate and an elevation in central venous pressure. This is followed by partial recovery of these parameters and then by a complete respiratory and cardiovascular collapse. Death appears to be due to a combination of peripheral vascular collapse and to an interruption in the normal respiratory mechanism. In addition, there appears to be some action of these venoms on the central nervous system of the experimental animals. This CNS effect is exhibited by a decrease in both the alpha and the beta rhythm of the brain (EEG). This change is also associated with a decrease in impulse transmission over the motor verves and progressive blockage of the neuromuscular apparatus which is similar to that produced by curare. Venoms had no effect on muscle response to direct stimulation.

A series of seven adult beagle dogs are used to study the effectiveness of cabenegrin I and II against the venoms. The dogs are anaesthetized with Na pentobarbital (30 mg/kg) and monitored for changes in arterial blood pressure, heart rate, electrocardiogram and respiration. Lethal doses (five to ten times of $LD_{50}$) of lyophilized reconstituted *Bothrops atrox* (Fer de Lance) (2.5 mg—5.0 mg/kg) or (10 mg/kg) South American rattle snake venom are administered.

Within 15 minutes following envenomation, marked decreases in heart rate and blood pressure are consistently noted. At from 15 to 30 minutes, respiration likewise decreases from an average of 20 per minute to 5 per minute. Treatment is initiated when severe cardiovascular embarrassment and apparent respiratory difficulties are observed, usually at from 15—30 minutes following envenomations.

A solution of cabenegrin I is prepared by dissolving 33 mg of the crystalline material of compound in 100 ml of aqueous ethanol (25:75). Similarly, a solution of 24 mg of cabenegrin II in 100 ml of aqueous ethanol (15:75) is prepared. Doses are prepared for administration by stirring 5 ml of each of the respective alcoholic solutions of the compounds of the invention into 50 ml of water.

Administration of the respective solutions is through a tube placed and advanced into the stomach of the dog. Treatment is as follows: No immediate response is noted following therapy. Blood pressure, heart rate and respiration all remain extremely low. At approximately 30 minutes following the first dose of the respective alcoholic solution of the compound of the invention, a slow gradual improvement of breathing occurs followed by partial restoration of heart rate and blood pressure. Continuous therapy is provided at 30 minute intervals in 50 ml water until all monitored vital signs return to within 10% of control. The effective dose range is between 10 and 20 ml of antivenom extract per animal. From two to four doses are required. After observation for 8—10 hours the animals are placed in a holding cage with food and water. At 24 hours, all dogs show signs of depressed activity. At 72 hours, all dogs are taking food and water. No additional therapy is required.

2 Activity of cabenegrin I and II against *E. coli* endotoxin.

Three adult beagle dogs are used to demonstrate the effectiveness of the cabenegrin I and II in treating shock caused by *E. coli* endotoxin. The dogs are anaesthetized with Na pentobarbital (30 mg/kg) and monitored for changes in arterial blood pressure, heart rate, EKG and respiration. Lethal doses (1 mg/kg) of *E. coli* endotoxin are injected i.v. into a catheter placed in the vein of the hind limb of the dogs. In the first experiment no antidotal therapy was initiated and the animal expired 2 hours after injection. In the three additional cases antidotal therapy is initiated at the time when severe cardiovascular collapse and respiratory difficulties appear. These usually occur within about 1½ hours after the injection of the toxin.

Solutions of cabenegrin I and II, prepared as described above, are administered by stomach tube in a single dose of 10 ml in 100 ml of water to each of the dogs. The animals so treated survive and resume normal activity.

3. Isolated heart (Langendorff) preparation

A series of 2 dog heart preparations are tested to measure the antivenom effect on coronary blood flow, heart rate, EKG, force of ventricular contraction and coronary vascular resistance when cabenegrin I and II are given either before or after lethal venom challenge. It is observed that treatment appears capable of overcoming the toxic effects of the venom on cardiovascular functions. These effects are a decrease of the force of contraction and heart rate. Coronary vascular resistance also increases progressively following the administration of venom. When solutions containing 0.05 mg/ml of either of the compounds of the invention are injected directly into the circulation prior to tropical rattle snake venom challenge, no detrimental effect on the heart is observed. Rather, the force of contraction and coronary blood flow increases by about 15 to 20%.

When either of the compounds is given following lethal challenge of tropical rattle snake venom, the antidote restores force of contraction and heart rate to normal levels and reverses the minor arrhythmias caused by envenomation.

4. Neurophysiological function

Three dogs and one cat are tested for the antidotal effect on neuromuscular function, action potential and brain wave activity following envenomation with lethal doses of Fer de Lance venom.

Snake venom decreases both brain wave activity and nerve impulse transmission. These are restored to nearly, if not completely, normal levels by the administration of cabenegrin I and II. Action potentials and neuromuscular function remain depressed for approximately 30 to 60 minutes after treatment with each of the compounds. This is followed by a slow, gradual return to control levels after from 12 to 24 hours.

In certain experiments in which complete neuromuscular blockage occurs and the animals are no longer capable of spontaneous respiration, artificial ventilation is required until the action of the compound has manifested itself. This may occur after envenomation, but once stabilized, the animals are capable of spontaneous breathing and no further therapy is required.

Cortical electrical activity is markedly (25—30%) depressed by the venom. These changes are restored to normal by the administration of the compounds cabenegrin I and II. Following treatment no further changes are noted.

Results of these studies indicate that oral or i.v. doses of cabenegrin I and II are capable of treating conditions clinically thought of as being either cardiotoxic and/or neurotoxic in nature with no inherent observable side effects.

As mentioned above, the new compounds of the method of the invention are suitable for the preparation of pharmaceutical compositions, preferably for oral administration, or for parenteral injections.

Suitable pharmaceutical carriers for oral administration include liquids which are inert to the gastric mucosa. Liquid carriers can be of the type in which a stable suspension of compounds of the invention can be prepared. Alternatively the liquid carrier can be a solvent for the cabenegrin I or II. In the latter case, the liquid pharmaceutical carrier solution can be prepared for either oral administration, or for parenteral injection.

Novel compositions for oral administration can also be prepared by blending cabenegrin I and II with appropriate dry pharmaceutical carriers known to the art. These dry compositions can be put into any suitable dosage form for ingestion including pills, tablets and capsules. Micro-encapsulation techniques can be employed to provide a sustained release of the desired dosage if the particular condition of the subject indicates this form of therapy.

In many instances, either the nature of the poisonous toxin, or the type of deteriorating condition of the subject will necessitate that a liquid dosage be administered to insure a prompt initiation of the therapeutic effects of the compounds. Effective treatment of animals or of subjects that are unconscious or whose vital functions are in an advanced state of deterioration will require administration of oral doses via stomach tube or intravenous injection by syringe or catheter.

A pharmaceutical composition was prepared by blending the following materials in the specified proportions by weight:

| | |
|---|---|
| Cabenegrin I | 20 |
| Starch | 15.0 |
| Magnesium stearate | 2.0 |
| Sodium benzoate | 6.0 |
| Benzalkonium chloride | 2.0 |

After the dry composition was thoroughly blended tablets were prepared from the mixture. Each tablet was formed so that it contained 100 mg of cabenegrin I. Similarly, tablets were prepared using the same mixture for the pharmaceutical carrier and the same proportion of cabenegrin II was substituted for the compound, with each tablet containing 70 mg of cabenegrin I.

**Claims for the Contracting States: BE CH DE FR GB IT LI LU NL SE**

1. A compound of the formula (I)

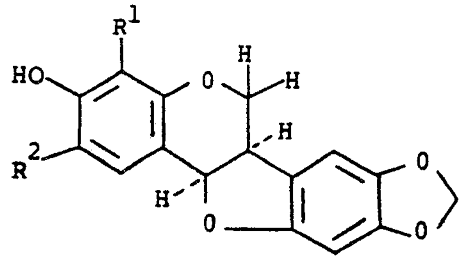

(I)

wherein $R^1$ represents a hydrogen atom and $R^2$ represents a $—CH_2CH_2—CH(CH_3)—CH_2OH$ group, or $R^1$ represents a

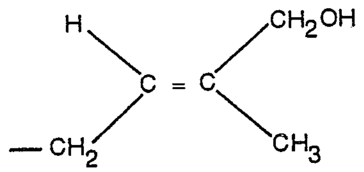

group

and $R^2$ represents a hydrogen atom, together with all optical isomers and racemic mixtures thereof.

2. A mixture of compounds of the formula (I) as defined in claim 1 wherein $R^2$ represents a 3-hydroxymethyl-but-1-yl group which comprises a 3:1 mixture of epimers at C-3′ of the butyl group.

3. A compound as claimed in claim 1 or mixture as claimed in claim 2 in crystalline form.

4. A compound or mixture as claimed in any of claims 1 to 3 wherein the $^1H$—NMR constants are:

1.83 (3H, d, 1 Hz);
3.40 (2H, br, d, 7 Hz);
3.40 (2H, ddd, 7, 7.5 Hz);
3.61 (2H, d, d, 11.7 Hz);
3.90 (2H);
3.99 (2H, br, s);
4.28 (1H, dd, 11.5 Hz);
5.45 (1H, br, t, 7 Hz);
5.45 (1H, d, 7 Hz);
6.41 (1H, s);
6.52 (1H, d, 9 Hz);
6.70 (1H, s);
7.23 (1H, d, 9 Hz) ppm

or

0.69 (3H, d, 5.8 Hz);
2.50 (2H, m);
2.67 (2H, m);
2.94 (1H, ddd, 9.1, 6.3, 4.9 Hz);
3.10 (2H, dd, 8.8, 2.7 Hz);
3.18 (2H, dd, 8.8, 4.4 Hz);
3.50 (1H, dd, 9.1, 9.1 Hz);
3.86 (1H, dd, 9.1, 4.9 Hz);
5.27 (1H, d, 6.3 Hz);
5.30 (2H);
5.33 (2H);
6.36 (1H);
6.52 (1H);
6.53 (1H);
7.08 (1H) ppm.

5. A process for the isolation of a compound of the formula (I)

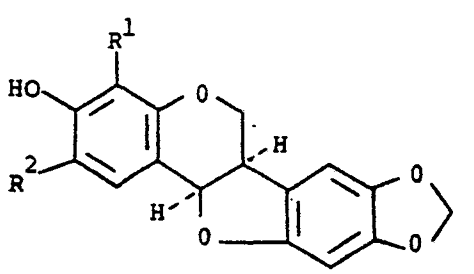

(I)

wherein $R^1$ and $R^2$ are as defined in claim 1 which comprises the steps of

a) treating the comminuted root of the cabeca de negra with a mixture of water and aliphatic alcohol having 1—4 carbon atoms then allowing to stand at ambient temperature until, after separating from the root, a solution having the colour of a strong tea is obtained;

b) concentrating said extract to provide an oily residue;

c) treating said oily residue with aqueous methanol and thereafter extracting with an aliphatic hydrocarbon having 5—8 carbon atoms;

d) extracting the remaining aqueous layer with ether; and

e) separating the ether layer which results by known chromatographic techniques to obtain a compound or compounds of formula (I).

6. A process as claimed in claim 5 which comprises extracting the oily residue obtained in step (c) with hexane.

7. A process as claimed in claim 5 or claim 6 which comprises subjecting the ether layer resulting from step (e) to high pressure liquid chromatography, eluting with methanol, and separating a first fraction and a second fraction and recovering from said first fraction a compound of formula (I) wherein $R^2$ is hydrogen and $R^1$ is a 3-hydroxymethylbut-2-en-1-yl group and from said second fraction a compound of formula (I) wherein $R^1$ is hydrogen and $R^2$ is a 3-hydroxymethyl-but-1-yl group.

8. A process as claimed in claim 7 wherein further purification is achieved either by subjecting the first fraction to high pressure liquid chromatography and eluting with methanol and methylene chloride and/or subjecting said second fraction to high pressure liquid chromatography and eluting with methanol, acetonitrile, water and n-propylalcohol.

9. A therapeutic composition comprising a compound of formula (I) or mixture of one or more thereof as defined in any of claims 1 to 4 in admixture with a pharmaceutically acceptable carrier, diluent or excipient.

10. A composition as claimed in claim 9 wherein the pharmaceutical carrier is a liquid and the composition is suitable either for oral or parenteral administration.

**Claims for the Contracting State: AT**

1. A process for the isolation of a compound of the formula (I)

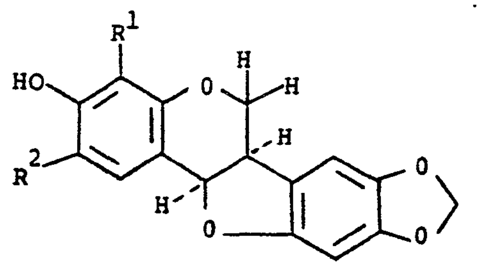

(I)

wherein $R^1$ represents a hydrogen atom and $R^2$ represents a $—CH_2CH_2—CH(CH_3)—CH_2OH$ group, or $R^1$ represents a

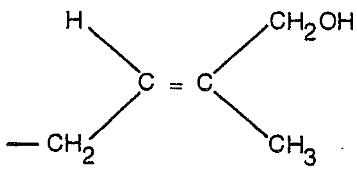 group

and $R^2$ represents a hydrogen atom, together with all optical isomers and racemic mixtures thereof, which comprises the steps of

a) treating the comminuted root of the cabeca de negra with a mixture of water and aliphatic alcohol having 1—4 carbon atoms then allowing to stand at ambient temperature until, after separating from the root, a solution having the colour of a strong tea is obtained;

b) concentrating said extract to provide the oily residue;

c) treating said oily residue with aqueous methanol and thereafter extracting with an aliphatic hydrocarbon having 5—8 carbon atoms;

d) extracting the remaining aqueous layer with ether; and

e) separating the ether layer which results by known chromatographic techniques to obtain a compound or compounds of formula (I).

2. A process as claimed in claim 1 which comprises extracting the oily residue obtained in step (c) with hexane.

3. A process as claimed in claim 1 or claim 2 which comprises subjecting the ether layer resulting from step (e) to high pressure liquid chromatography, eluting with methanol, and separating a first fraction and a second fraction and recovering from said first fraction a compound of the formula (I) wherein $R^2$ is hydrogen and $R^1$ is a 3-hydroxymethylbut-2-en-1-yl group and from said second fraction a compound of formula (I) wherein $R^1$ is hydrogen and $R^2$ is a 3-hydroxymethyl-but-1-yl group.

4. A process as claimed in claim 7 wherein further purification is achieved either by subjecting the first fraction to high pressure liquid chromatography and eluting with methanol and methylene chloride and/or subjecting said second fraction to high pressure liquid chromatography and eluting with methanol, acetonitrile, water and n-propylalcohol.

5. A process as claimed in any of claims 1 to 4 wherein the compound of formula (I) obtained is a compound wherein $R^2$ represents a 3-hydroxymethyl-but-1-yl group which comprises a 3:1 mixture of epimers at C-3′ of the butyl group.

6. A process as claimed in claims 1 to 5 wherein the compound of formula (I) is obtained in crystalline form.

7. A process as claimed in any of claims 1 to 6 wherein the compound of formula (I) obtained is a compound wherein the $^1H$—NMR constants are:

1.83 (3H, d, 1 Hz);
3.40 (2H, br, d, 7 Hz);
3.40 (2H, ddd, 7, 7.5 Hz);
3.61 (2H, d, d, 11.7 Hz);
3.90 (2H);
3.99 (2H, br, s);
4.28 (1H, dd, 11.5 Hz);
5.45 (1H, br, t, 7 Hz);
5.45 (1H, d, 7 Hz);
6.41 (1H, s);
6.52 (1H, d, 9 Hz);
6.70 (1H, s);
7.23 (1H, d, 9 Hz) ppm

or

0.69 (3H, d, 5.8 Hz);
2.50 (2H, m);
2.67 (2H, m);
2.94 (1H, ddd, 9.1, 6.3, 4.9 Hz);
3.10 (2H, dd, 8.8, 2.7 Hz);
3.18 (2H, dd, 8.8, 4.4 Hz);
3.50 (1H, dd, 9.1, 9.1 Hz);
3.86 (1H, dd, 9.1, 4.9 Hz);
5.27 (1H, d, 6.3 Hz);
5.30 (2H);
5.33 (2H);
6.36 (1H);
6.52 (1H);
6.53 (1H);
7.08 (1H) ppm.

8. A process for the preparation of a therapeutic composition comprising a compound of formula (I) or mixture of one or more thereof as defined in any of claims 1 to 4 in admixture with a pharmaceutically acceptable carrier, diluent or excipient.

9. A process as claimed in claim 8 wherein the pharmaceutical carrier is a liquid and the composition thus prepared is suitable either for oral or parenteral administration.

**Patentansprüche für die Vertragsstaaten: BE CH DE FR GB IT LI LU NL SE**

1. Verbindung der Formel (I)

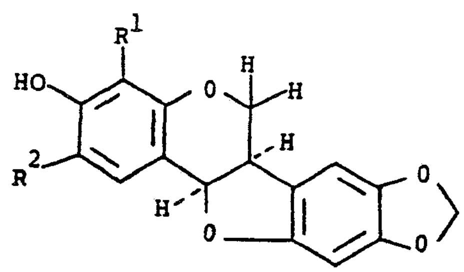 (I)

worin $R^1$ ein Wasserstoffatom bedeutet, und $R^2$ eine $—CH_2CH_2—CH(CH_3)—CH_2OH—$Gruppe darstellt, oder $R^1$ eine

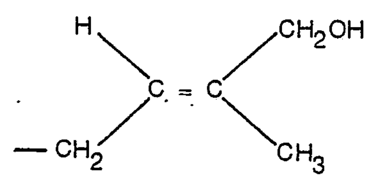 Gruppe

bedeutet, und $R^2$ ein Wasserstoffatom darstellt, zusammen mit sämtlichen optischen Isomeren und razemischen Gemischen derselben.

2. Gemisch der Verbindungen der Formel (I) gemäss Anspruch 1, worin $R^2$ eine 3-Hydroxymethyl-but-1-yl-Gruppe bedeutet, welche ein 3:1-Gemisch von Epimeren am C-3′ der Butylgruppe umfasst.

3. Verbindung nach Anspruch 1 oder Gemisch gemäss Anspruch 2 in kristalliner Form.

4. Verbindung oder Gemisch gemäss einem oder mehreren der Ansprüche 1 bis 3, mit folgenden $^1H$—NMR-Konstanten:

1,83 (3H, d, 1Hz);
3,40 (2H, br, d, 7 Hz);
3,40 (2H, ddd, 7, 7,5 Hz);
3,61 (2H, d, d, 11,7 Hz);
3,90 (2H);
3,99 (2H, br, s);
4,28 (1H, dd, 11,5 Hz);
5,45 (1H, br, t, 7 Hz);
5,45 (1H, d, 7 Hz);
6,41 (1H, s);
6,52 (1H, d, 9 Hz);
6,70 (1H, s);
7,23 (1H, d, 9 Hz) ppm

oder

0,69 (3H, d, 5,8 Hz);
2,50 (2H, m);
2,67 (2H, m);
2,94 (1H, ddd, 9,1, 6,3, 4,9 Hz);
3,10 (2H, dd, 8,8, 2,7 Hz);
3,18 (2H, dd, 8,8, 4,4 Hz);
3,50 (1H, dd, 9,1, 9,1 Hz);
3,86 (1H, dd, 9,1, 4,9 Hz);
5,27 (1H, d, 6,3 Hz);
5.30 (2H);
5,33 (2H);
6,36 (1H);
6.52 (1H);
6,53 (1H);
7,08 (1H) ppm.

5. Verfahren zur Isolierung einer Verbindung der Formel (I)

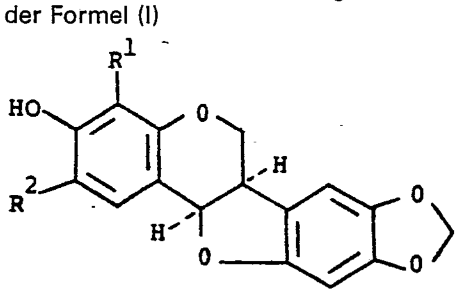

(I)

worin $R^1$ und $R^2$ die in Anspruch 1 angegebene Bedeutung haben, gekennzeichnet durch die folgenden Schritte:

a) Behandeln der zerkleinerten Wurzel von Cabeca-de-negra mit einem Gemisch aus Wasser und aliphatischem Alkohol mit 1 bis 4 Kohlenstoffatomen, Stehenlassen bei Raumtemperatur bis nach dem Abscheiden aus der Wurzel eine Lösung mit der Farbe von starkem Tee erhalten wird;

b) Konzentrieren des genannten Extraktes zu einem öligen Rückstand;

c) Behandeln des genannten öligen Rückstandes mit wässrigem Methanol und anschliessendes Extrahieren mit einem aliphatischen Kohlenwasserstoff mit 5 bis 8 Kohlenstoffatomen;

d) Extrahieren der restlichen wässrigen Schicht mit Ether; und

3) Abtrennen der Etherschicht und Erhalt einer Verbindung oder Verbindungen der Formel (I) mittels bekannter chromatografischer Methoden.

6. Verfahren nach Anspruch 5, dadurch gekennzeichnet, dass es ein Extrahieren des in Schritt c) erhaltenen Rückstandes mit Hexan umfasst.

7. Verfahren nach Anspruch 5 oder 6, dadurch gekennzeichnet, dass die aus Schritt e) resultierende Etherschicht einer Hochdruck-Flüssigchromatografie unterworfen wird, Eluieren mit Methanol und Abtrennen einer ersten Fraktion und einer zweiten Fraktion, und Gewinnung einer Verbindung der Formel (I) aus der genannten ersten Fraktion, worin $R^2$ Wasserstoff darstellt und $R^1$ eine 3-Hydroxymethyl-but-2-en-yl-Gruppe bedeutet, und einer Verbindung der Formel (I) aus der genannten zweiten Fraktion, worin $R^1$ Wasserstoff darstellt und $R^2$ eine 3-Hydroxymethyl-but-1-yl-Gruppe bedeutet.

8. Verfahren nach Anspruch 7, worin eine weitere Reinigung erzielt wird, entweder indem die erste Fraktion einer Hochdruck-Flüssigchromatografie unterworfen und mit Methanol und Methylenchlorid eluiert wird und/oder indem die genannte zweite Fraktion einer Hochdruck-Flüssigchromatografie unterworfen und mit Methanol, Acetonitril, Wasser und n-Propylakohol eluiert wird.

9. Therapeutische Zubereitung, gekennzeichnet durch eine Verbindung der Formel (I) oder ein Gemisch aus einer oder mehreren derselben, wie sie in den Ansprüchen 1 bis 4 definiert sind, zusammen mit einem pharmazeutisch annehmbaren Träger, Verdünnungsmittel oder Excipienten.

10. Zubereitung nach Anspruch 9, worin der pharmazeutische Träger eine Flüssigkeit darstellt und die Zubereitung entweder zur oralen oder parenteralen Verabreichnung geeignet ist.

**Patentansprüche für den Vertragsstaat: AT**

1. Verfahren zur Isolierung einer Verbindung der Formel (I)

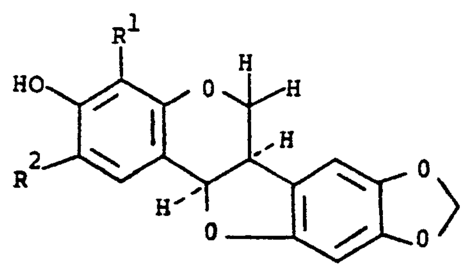

(I)

worin $R^1$ ein Wasserstoffatom bedeutet, und $R^2$ eine $—CH_2CH_2—CH(CH_3)—CH_2OH—$Gruppe darstellt, oder

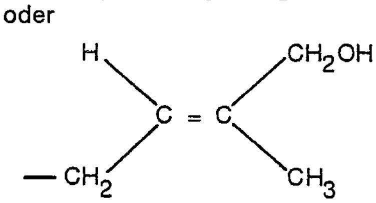

Gruppe

bedeutet, und $R^2$ ein Wasserstoffatom darstellt, zusammen mit sämtlichen optischen Isomeren und razemischen Gemischen derselben, gekennzeichnet durch die folgenden Schritte:

a) Behandeln der zerkleinerten Wurzel von Cabeca-de-negra mit einem Gemisch aus Wasser und aliphatischem Alkohol mit 1 bis 4 Kohlenstoffatomen, Stehenlassen bei Raumtemperatur bis nach dem Abscheiden aus der Wurzel eine Lösung mit der Farbe von starkem Tee erhalten wird;

b) Konzentrieren des genannten Extraktes zu einem öligen Rückstand;

c) Behandeln des genannten öligen Rückstandes mit wässrigem Methanol und anschliessendes Extrahieren mit einem aliphatischen Kohlenwasserstoff mit 5 bis 8 Kohlenstoffatomen;

d) Extrahieren der restlichen wässrigen Schicht mit Ether; und

e) Abtrennen der Etherschicht und Erhalt einer Verbindung oder Verbindungen der Formel (I) mittels bekannter chromatografischer Methoden.

2. Verfahren nach Anspruch 1, dadurch gekennzeichnet, dass es ein Extrahieren des in Schritt c) erhaltenen Rückstandes mit Hexan umfasst.

3. Verfahren nach Anspruch 2 oder 3, dadurch gekennzeichnet, dass die aus Schritt e) resultierende Etherschicht einer Hochdruck-Flüssigchromatografie unterworfen wird, Eluieren mit Methanol und Abtrennen einer ersten Fraktion und einer zweiten Fraktion, und Gewinnung einer Verbindung der Formel (I) aus der genannten ersten Fraktion, worin $R^2$ Wasserstoff darstellt und $R^1$ eine 3-Hydroxymethyl-but-2-en-yl-Gruppe bedeutet, und einer Verbindung der Formel (I) aus der genannten zweiten Fraktion, worin $R^1$ Wasserstoff darstellt und $R^2$ eine 3-Hydroxymethyl-but-1-yl-Gruppe bedeutet.

4. Verfahren nach Anspruch 3, worin eine weitere Reinigung erzielt wird, entweder indem die erste Fraktion einer Hockdruck-Flüssigchromatografie unterworfen und mit Methanol und Methylenchlorid eluiert wird und/oder indem die genannte zweite Fraktion einer Hochdruck-Flüssigchromatografie unterworfen und mit Methanol, Acetonitril, Wasser und n-Propylakohol eluiert wird.

5. Verfahren nach einem oder mehreren der Ansprüche 1 bis 4, worin die erhaltene Verbindung der Formel (I) eine Verbindung darstellt, in welcher $R^2$ eine 3-Hydroxymethyl-but-1-yl-Gruppe bedeutet, die ein 3:1-Gemisch von Epimeren am C-3' der Butylgruppe umfasst.

6. Verfahren nach Anspruch 1 bis 5, worin die Verbindung der Formel (I) in kristalliner Form erhalten wird.

7. Verfahren nach einem oder mehreren der Ansprüche 1 bis 6, worin die erhaltene Verbindung der Formel (I) eine Verbindung darstellt, in welcher die $^1$H-NMR-Konstanten betragen:

1,83 (3H, d, 1 Hz);
3,40 (2H, br, d, 7 Hz);
3,40 (2H, ddd, 7, 7,5 Hz);
3,61 (2H, d, d, 11,7 Hz);
3,90 (2H);
3,99 (2H, br, s);
4,28 (1H, dd, 11,5 Hz);
5,45 (1H, br, t, 7 Hz);
5,45 (1H, d, 7 Hz);
6,41 (1H, s);
6,52 (1H, d, 9 Hz);
6,70 (1H, s);
7,23 (1H, d, 9 Hz) ppm

oder

0,69 (3H, d, 5,8 Hz);
2,50 (2H, m);
2,67 (2H, m);
2,94 (1H, ddd, 9,1, 6,3, 4,9 Hz);
3,10 (2H, dd, 8,8, 2,7 Hz);
3,18 (2H, dd, 8,8, 4,4 Hz);
3,50 (1H, dd, 9,1, 9,1 Hz);
3,86 (1H, dd, 9,1, 4,9 Hz);
5,27 (1H, d, 6,3 Hz);
5,30 (2H);
5,33 (2H);
6,36 (1H);
6,52 (1H);
6,53 (1H);
7,08 (1H) ppm.

8. Verfahren zur Herstellung einer therapeutischen Zubereitung, welche eine Verbindung der Formel (I) oder ein Gemisch einer oder mehrerer derselben, wie sie in den Ansprüchen 1 bis 4 definiert sind, zusammen mit einem pharmazeutisch annehmbaren Träger, Verdünnungsmittel oder Excipienten umfasst.

9. Verfahren nach Anspruch 8, worin der pharmazeutische Träger eine Flüssigkeit darstellt und die derart hergestellte Zubereitung entweder zur oralen oder zur parenteralen Verabreichung geeignet ist.

**Revendications pour les Etats contractants: BE CH DE FR GB IT LI LU NL SE**

1. Composé de formule (I)

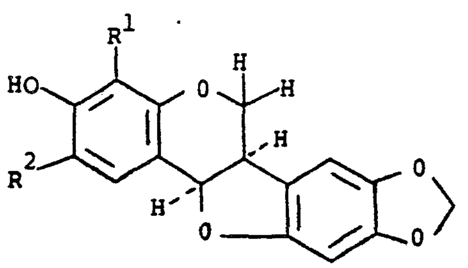

(I)

dans laquelle $R^1$ représente un atome d'hydrogène et $R^2$ représente un groupe $—CH_2CH_2—CH(CH_3)—CH_2OH$, ou $R^1$ représente un groupe

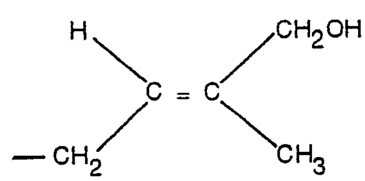

et $R^2$ représente un atome d'hydrogène, ainsi que tous ses isomères optiques et mélanges racémiques.

2. Mélange des composés de formule (I) selon la revendication 1, où $R^2$ représente un groupe 3-hydroxyméthyl-but-1-yle caractérisé en ce qu'il comprend un mélange 3:1 des épimères au niveau du C-3' du groupe butyle.

3. Composé selon la revendication 1 ou mélange selon la revendication 2 caractérisé en ce qu'il est sous une forme cristalline.

4. Composé ou mélange selon l'une quelconque des revendications 1 à 3, caractérisé en ce que les constantes de RMN-$^1$H sont:

1,83 (3H, d, 1 Hz);
3,40 (2H, large, d, 7 Hz);
3,40 (2H, ddd, 7, 7,5 Hz);
3,61 (2H, d, d, 11,7 Hz);
3,90 (2H);
3,99 (2H, large, s);
4,28 (1H, dd, 11,5 Hz);
5,45 (1H, large, t, 7 Hz);
5,45 (1H, d, 7 Hz);
6,41 (1H, s);
6,52 (1H, d, 9 Hz);
6,70 (1H, s);
7,23 (1H, d, 9 Hz) ppm

ou

0.69 (3H, d, 5,8 Hz);
2,50 (2H, m);
2,67 (2H, m);
2,94 (1H, ddd, 9,1, 6,3, 4,9 Hz);
3,10 (2H, dd, 8,8, 2,7 Hz);
3,18 (2H, dd, 8,8, 4,4 Hz);
3,50 (1H, dd, 9,1, 9,1 Hz);
3,86 (1H, dd, 9,1, 4,9 Hz);
5,27 (1H, d, 6,3 Hz);
5,30 (2H);
5,33 (2H);
6,36 (1H);
6,52 (1H);
6,53 (1H);
7,08 (1H) ppm.

5. Procédé pour l'isolement d'un composé de formule (I)

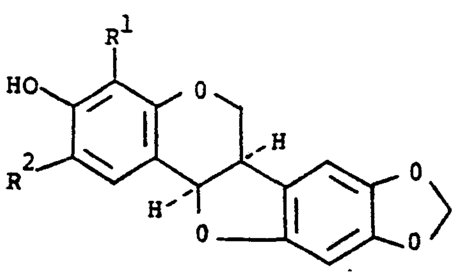

(I)

dans laquelle $R^1$ et $R^2$ sont tels que définis dans la revendication 1, caractérisé en ce qu'il comporte les étapes qui consitent

a) à traiter la racine du "cabeca de negra" reduite en poudre avec un mélange d'eau et d'alcool aliphatique ayant 1—4 atomes de carbone, puis à les laisser au repos à la température ambiante jusqu'à ce que l'on obtienne, après séparation de la racine, une solution ayant la couleur d'un thé fort;

b) à concentrer ledit extrait pour obtenir un résidu huileux;

c)à traiter ledit résidu huileux avec du méthanol aqueux et ensuite à l'extraire avec un hydrocarbure aliphatique ayant 5—8 atomes de carbone;

d) à extraire la couche aqueuse restante avec de l'éther; et

e) à séparer la couche d'ether qui en résulte par des techniques chromatographiques connues pour obtenir un composé ou des composés de formule (I).

6. Procédé selon la revendication 5, caractérisé en ce qu'il comprend l'extraction à l'hexane du résidu huileux obtenu dans l'étape (c).

7. Procédé selon l'une quelconque des revendications 5 ou 6, caractérisé en ce qu'il consiste à soumettre la couche d'éther provenant de l'étape (e) à une chromatographie liquide à haute pression, à l'éluer avec du méthanol, et à séparer une première fraction et une seconde fraction et à récupérer à partir de ladite première fraction un composé de formule (I) dans laquelle $R^2$ est l'hydrogène et $R^1$ est un groupe 3-hydroxyméthyl-but-2-en-1-yle et à partir de ladite seconde fraction, un composé de formule (I) dans laquelle $R^1$ est l'hydrogène et $R^2$ est un groupe 3-hydroxyméthyl-but-1-yle.

8. Procédé selon la revendication 7, caractérisé en ce que l'on obtient une purification supplémentaire en soumettant la première fraction a une chromatographie liquide à haute pression et en l'éluant avec du méthanol et du chlorure de méthylène et/ou en soumettant ladite seconde fraction à une chromatographie liquide à haute pression et en l'éluant avec du méthanol, de l'acétonitrile, de l'eau et de l'alcool n-propylique.

9. Composition thérapeutique comprenant un composé de formule (I) ou un mélange d'un ou plusieurs des produits selon l'une quelconque des revendications 1 à 4, en mélange avec un véhicule, un diluant ou un excipient pharmaceutiquement acceptable.

10. Composition selon la revendication 9, caractérisée en ce que le véhicule pharmaceutique est un liquide et en ce que la composition convient pour l'administration soit par voie orale, soit par voie parentérale.

**Revendications pour l'Etat contractant: AT**

1. Procédé pour l'isolement d'un composé de formule (I)

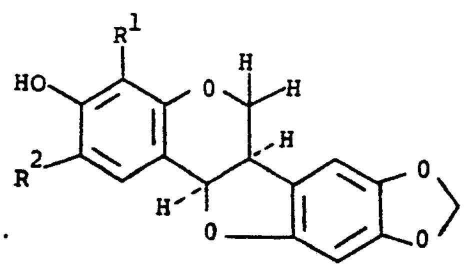

(I)

dans laquelle $R^1$ représente un atome d'hydrogène et $R^2$ représente un groupe $-CH_2CH_2-CH(CH_3)-CH_2OH$, ou $R^1$ représente un groupe

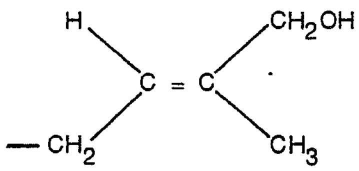

et $R^2$ représente un atome d'hydrogène, ainsi que tous ses isomères optiques et mélanges racémiques,caractérisé en ce qu'il comporte les étapes qui consistent

a) à traiter la racine du "cabeca de negra" reduite en poudre avec un mélange d'eau et d'alcool aliphatique ayant 1—4 atomes de carbone, puis à les laisser au repos à la température ambiante jusqu'à ce que l'on obtienne, après séparation de la racine, une solution ayant la couleur d'un thé fort;

b) à concentrer ledit extrait pour obtenir un résidu huileux;

c) à traiter ledit résidu huileux avec du méthanol aqueux et ensuite à l'extraire avec un hydrocarbure aliphatique ayant 5—8 atomes de carbone;

d) à extraire la couche aqueuse restante avec de l'ether; et

e) à séparer la couche d'éther qui en résulte par des techniques chromatographiques connues pour obtenir un composé ou des composés de formule (I)

2. Procédé selon la revendicatioon 1, caractérisé en ce qu'il comprend l'extraction à l'hexane du résidu huileux obtenu dans l'étape (c).

3. Procédé selon l'une quelconque des revendications 1 ou 2, caractérisé en ce qu'il consiste à soumettre la couche d'éther provenant de l'étape (e) à une chromatographie liquide à haute pression, à l'éluer avec du méthanol, et à séparer une première fraction et une seconde fraction et à récupérer à partir de ladite première fraction un composé de formule (I) dans laquelle $R^2$ est l'hydrogène et $R^1$ est un groupe 3-hydroxyméthyl-but-2-en-l-yle et à partir de ladite seconde fraction, un composé de formule (I) dans laquelle $R^1$ est l'hydrogène et $R^2$ est un groupe 3-hydroxyméthyl-but-1-yle.

4. Procédé selon la revendication 7, caractérisé en ce que l'on obtient une purification supplémentaire en soumettant la première fraction à une chromatographie liquide à haute pression et en l'éluant avec du méthanol et du chlorure de méthylène et/ou en soumettant ladite seconde fraction à une chromatographie liquide à haute pression et en l'éluant avec du méthanol, de l'acétonitrile, de l'eau et de l'alcool n-propylique.

5. Procédé selon l'une quelconque des revendications 1 à 4 caractérisé en ce que le composé de formule (I) obtenu est un composé dans lequel $R^2$ représente un groupe 3-hydroxyméthyl-but-1-yle qui comprend un mélange 3:1 des épimères au niveau du C-3' du butyle.

6. Procédé selon l'une quelconque des revendications 1 à 5, caractérisé en ce que le composé de formule (I) est obtenu sous une forme cristalline.

7. Procédé selon l'une quelconque des revendications 1 à 6, caractérisé en ce que le composé de formule (I) obtenu est un composé dans lequel les constantes de RMN—$^1$H sont:

1,83 (3H, d, 1 Hz);
3,40 (2H, large, d, 7 Hz);
3.40 (2H, ddd, 7, 7,5 Hz);
3,61 (2H, d, d, 11,7 Hz);
3,90 (2H);
3,99 (2H, large, s);
4,28 (1H, dd, 11,5 Hz);
5,45 (1H, large, t, 7 Hz);
5,45 (1H, d, 7 Hz);
6,41 (1H, s);
6,52 (1H, d, 9 Hz);
6,70 (1H, s);
7,23 (1H, d, 9 Hz) ppm

ou

0.69 (3H, d, 5,8 Hz);
2,50 (2H, m);
2,67 (2H, m);
2,94 (1H, ddd, 9,1, 6,3, 4,9 Hz);
3,10 (2H, dd, 8,8, 2,7 Hz);
3,18 (2H, dd, 8,8, 4,4 Hz);
3,50 (1H, dd, 9,1, 9,1 Hz);
3,86 (1H, dd, 9,1, 4,9 Hz);
5,27 (1H, d, 6,3 Hz);
5.30 (2H);
5.33 (2H);
6,36 (1H);
6,52 (1H);
6,53 (1H);
7,08 (1H) ppm.

8. Procédé pour la préparation d'une composition thérapeutique comprenant un composé de formule (I) ou un mélange d'un ou plusieurs des produits selon l'une quelconque des revendications 1 à 4, en mélange avec une véhicule, un diluant, ou un excipient pharmaceutiquement acceptable.

9. Procédé selon la revendication 8, caractérisé en ce que le véhicule pharmaceutique est un liquide et en ce que la composition ainsi préparée convient pour l'administration soit par voie orale, soit par voie parentérale.